# EUROPEAN PATENT SPECIFICATION

(11) **EP 1 227 826 B1**
(45) Date of publication and mention of the grant of the patent: **24.09.2003**
(21) Application number: 00960126.1
(22) Date of filing: 06.07.2000
(51) Int. Cl.: A61K 35/56, A61P 29/00, A61P 37/02

(54) **COMPOSITION COMPRISING AN EXTRACT OF PERNA CANALICULUS, METHYLSULFONYLMETHANE AND GLUCOSAMINE AND USE THEREOF**
ZUSAMMENSETZUNG ENTHALTEND EINEN EXTRAKT AUS PERNA CANALICULUS, METHYLSULFONYLMETHAN UND GLUCOSAMINE UND VERWENDUNG
COMPOSITIONS COMPRENANT UN EXTRAIT DE PERNA CANALICULUS, DU METHYLSULFONYLMETHANE ET DE LA GLUCOSAMINE AINSI QUE LEUR UTILISATION

(30) Priority: 06.07.1999 US 142392 P
(43) Date of publication of application: 07.08.2002
(73) Proprietor: Foodscience Corporation, Essex Junction, VT 05453 (US)
(72) Inventor: KENDALL, Roger, V., Westford, VT 05494 (US)
(74) Representative: Dey, Michael, Dr.
(86) International application number: US0040298
(87) International publication number: WO01001976

(56) References cited:
- EP-A- 0 982 034
- WO-A-00/64436
- MELETIS: "natural relief for inflammation of sprains..." ALTERNATIVE AND COMPLEMENTARY THERAPIES, vol. 6, no. 3, pages 141-144, XP000997251
- DATABASE WPI Section Ch, Week 200102 Derwent Publications Ltd., London, GB; Class B05, AN 2001-016556 XP002165457 & ZA 9 905 557 A (LOG-NEGENTIEN BELEGGINGS PTY LTD), 28 June 2000 (2000-06-28)
- MOORE R D ET AL: "DIMINISHED INFLAMMATORY JOINT DISEASE IN MRL-1PR MICE INGESTING DIMETHYL SULFOXIDE OR METHYLSULFONYLMETHANE" FEDERATION PROCEEDINGS, vol. 44, no. 3, 1985, page 530 XP000997358 ISSN: 0014-9446
- DATABASE WPI Section Ch, Week 199927 Derwent Publications Ltd., London, GB; Class B04, AN 1999-325689 XP002165458 & NZ 314 867 A (MCFARLANE LAB NZ LTD), 29 April 1999 (1999-04-29) & GB 2 347 349 A 6 September 2000 (2000-09-06)

## Description

### BACKGROUND OF INVENTION

This invention relates to the use of methylsulfonylmethane and glucosamine, or salts thereof, in combination with at least one component derived from *Perna canaliculus* for the manufacture of a medicament for the support, regeneration and repair of connective tissues, in both animals and humans.

The use of preparations made from *Perna canaliculus* for therapeutic effect extends back at least twenty-five years since the mid-seventies. The nutritional and therapeutic properties of freeze-dried *Perna canaliculus* in alleviating the symptoms of arthritis were reported in Croft, 1979, *Relief From Arthritis* (Thorsons Publishing Group, Rochester, Vermont). Studies in both animal and human experiments have given mixed results, but indicate overall that certain components in the mussels were potentially helpful in relieving inflammation and pain associated with arthritis. Certain of these studies are as follows: McFarlane, June 1975, *New Zealand Medical Journal*, pg. 569 (the results of a study of the effects of a *Perna canaliculus* product called "Seatone" on a small group of arthritic individuals were reported); Rainsford and Whitehouse, 1980, *Arzeim-Forsch*, pg. 2128-2131 (a freeze-dried powdered preparation of whole mussel which was given orally to rats showed some modest anti-inflammatory activity in an induced rheumatoid arthritis model); Miller and Ormrod, 1980, *The New Zealand Medical Journal* 92:187 (a crude fraction of *Perna canaliculus* had a marked anti-inflammatory effect when administered by intra peritoneal injection in rats); Couch et al., 1982, *The New Zealand Medical Journal* 95:803 (a protein containing fraction of *Perna canaliculus* had an anti-inflammatory effect); Caughey et al., 1983, *European Journal of Rheumatology and Inflammation* 6:197 (in a human study involving forty-seven rheumatoid arthritic patients, no improvement was observed in patients given mussel extract as compared to patients taking the anti-inflammatory drug naproxen); Gibson et al., 1980, *Practitioner* 224:955 (93:111 (in human trials for both osteoarthritis and rheumatoid arthritis, significant benefits were achieved after three months on a *Perna canaliculus* preparation, including reduced pain and stiffness and improvement on functional tests); Audeval and Bouchart, 1986, *Gazette Medicale* 93:111(in human trials for osteoarthritis, forty percent of subjects showed significant improvement after treatment with a *Perna canaliculus* preparation); Miller et al., 1993, *Agents Actions* 38:139 (an aqueous fraction of *Perna canaliculus* inhibited experimentally induced inflammation in rats); Whitehouse et al., 1997, *Inflammopharmacology* (a lipid extract of *Perna canaliculus* exhibited a dose related anti-inflammatory activity).

Glucosamine and glucosamine sulfate are amino sugars which are present in the proteoglycans and glycosaminoglycans of joints and connective tissues and in the proteoglycans and glycosaminoglycans of basement membranes, mucous membranes and synovial fluids. A deficiency or the accelerated loss of these building blocks can lead to a decrease of synthesis of these important macromolecules during times of stress injury or trauma.

Methylsulfonylmethane (MSM) is naturally found in body tissues and can serve as an important source of biological sulfur.

Some of the most difficult health problems facing medical doctors and veterinarians are those areas dealing with autoimmune disorders, inflammatory conditions and connective tissue and joint diseases. Connective tissues of animals and humans, including, for example, the joints, tendons, ligaments and synovial fluids, are subject to degeneration as a result of stress, repetitive motion, and strains that can lead to the development of osteoarthritis. Rheumatoid arthritis, an autoimmune disease, can also lead to the connective tissue destruction, and pain and afflication, although the etiology is different. Prevention and treatment of such conditions is a major challenge because of the myriad of factors which affect connective tissue disorders. At present much of the therapeutic response focuses on reducing the symptoms (pain, inflammation, and reduced flexibility) rather than dealing with the fundamental cause of the disorders. Drugs such as corticosteroids and other non-steroidal anti-inflammatory drugs (NSAIDS), such as aspirin and carprofen, are frequently used. Such products give short term relief from swelling and pain, but have serious side-effects in the long run and interfere and block the body's own natural mechanisms to restore joint health.

### SUMMARY OF THE INVENTION

In accordance with this invention, it has been discovered that methylsulfonylmethane (MSM) and glucosamine, or salts thereof, in combination with at least one component derived from *Perna canaliculus* (referred to herein as *Perna canaliculus* extract (PCE)) is unexpectedly effective for the support, regeneration, and repair of connective tissues in both animals and humans. In particular, the maintenance and support of joints and connective tissues subject to stress, wear and tear, and disease processes as a result of inflammatory conditions, autoimmune disorders, and connective tissue and joint diseases is contemplated. In particular, synergistic effects of the above combination in the treatment of joint and connective tissue conditions is contemplated. The synergistic combination modulates immune and inflammatory responses and promotes enhanced regeneration of the constituents of the affected joints and tissues. An object of the present invention is to provide protection and repair of connective tissues of both animals and humans.

Treatment, as used herein, pertains to the therapeutic administration of the compounds of the invention for the prevention, amelioration, or cure of disease.

The treatment of both humans and animals is contemplated Animals are preferably vertebrate animals, preferably mammals, including but not limited to dogs, cats, horses, cows, pigs etc., including other household pets and farm animals.

Connective tissues, as used herein, includes, but is not limited to joints, cartilage tendons, ligaments, synovial fluids, collagen, and proteoglycans.

Conditions that may be treated in accordance with this invention include, for example, potential or existing joint or connective tissue conditions or inflammatory conditions. Inflammatory conditions include, for example, systemic autoimmune diseases such as, for example, lupus erythematosus, rheumatoid arthritis, and multiple sclerosis, and organ specific autoimmune diseases such as, for example, myasthenia gravis, Grave's disease, Hashimoto's thyroiditis, Crohn's disease, autoimmune hemolytic anemias, insulin dependent diabetes mellitus, glomerulonephritis, and rheumatic fever. Other diseases that may be treated in accordance with this invention include arthritic conditions such as osteoarthritis and gouty arthritis, as well as other inflammatory conditions such as conjunctivitis, dermatitis, bronchitis, rhinitis etc., brought about by injury, allergies, infections, microorganisms, trauma, or physical or chemical agents. Osteoarthritis, as used herein, refers to both primary osteoarthritis, which is not related to trauma or disease in the joint, and secondary osteoarthritis, which is associated with alterations in articular structure. Secondary osteoarthritis is far more common than primary osteoarthritis and can be induced by some form of trauma, such as fracture, sprain, etc., and by inflammatory response and/or metabolic, endocrine, neuropathic or drug induced responses.

Additionally, the treatment of inflammatory aspects of asthma is also contemplated.

One aspect of the invention is a composition comprising a combination of MSM, glucosamine or salts thereof, and at least one PCE component. The PCE component may be any therapeutically active component derived from the flesh of the mussel or its organs, which are suitable for use in the preparation of food supplements or pharmaceutical preparations. The invention contemplates the use of unrefined components of the mussel, such as whole mussel, or any therapeutically active extracts thereof. As used herein, a therapeutically active PCE component according to this invention is a component that is effective in reducing inflammation in animals or humans or that has immunomodulating effects in humans or animals. For example, several animal models for arthritis that are well known in the art may be used to determine therapeutic activity of a PCE component. Such models include, for example, collagen induced arthritis in rats or mice, carrageenan-induced inflammatory oedema in rats as described in Miller et al., 1980, *New Zealand Medical Journal* 92:667, and arthritis induced in rats and mice by the injection of adjuvants prepared from dried *Mycobacterium tuberculosis* as described in Whitehouse, et al., 1997, *Inflammopharmacology* 5:237-246. Therapeutic activity of a PCE component may also be determined by evaluating whether the PCE component is effective for reducing anti-nuclear antibodies, particularly anti-ssDNA antibodies or anti-dsDNA antibodies, in humans with SLE or in the mouse model for SLE, as described in U.S. Patent Application Serial No. 09/316,001 (Kendall and Lawson). In particular, the PCE may be a preparation of whole mussel which has been freeze-dried and ground such as, for example, the *Sea Mussel* product available from FoodScience Corporation, Essex Junction, Vermont or the *Perna* product available from DaVinci Laboratories, Essex Junction, Vermont. Additionally, therapeutically active extracts of *Perna canaliculus* may also be employed. Such therapeutically active extracts have been described as follows: Macrides and Kalafatis, PCT/AU95/00485, WO 96/05164 (a purified lipid extract); Whitehouse et al., 1997, *Inflammopharmacology* 5:237-246 (a purified lipid extract); Kosuge and Sugiyama, U.S. Patent No. 4,801,453 (stabilized mussel extract); Couch et al., 1982, *The New Zealand Medical Journal* 95:803 (a protein fraction); Miller et al., 1993, *Agents Actions* 38:139 (an aqueous fraction).

The glucosamine component may be glucosamine, or a pharmacologically acceptable salt thereof, such as glucosamine sulfate, for example. A composition containing MSM, glucosamine, and PCE may be formulated as a pharmaceutical composition or as a dietary supplement using techniques that are well known in the art. A preferred formulation of the composition is a formulation for oral administration.

Another aspect of this invention is the use of a combination of a glucosamine component, an MSM component, and at least one PCE component for the manufacture of a medicament for treating potential or existing joint or connective tissue conditions, or inflammatory conditions.

The MSM, glucosamine and PCE components may be administered via the same route or they may be administered via different routes. For example, the MSM, glucosamine, and PCE may all be administered orally, either simultaneously or at different times. A preferred route for administration of PCE, particularly ground whole mussel, is via oral administration. A preferred route for the administration of MSM or glucosamine is also is via oral administration. MSM, PCE and glucosamine can also be administered by intramuscular injection, intra peritoneal injection, parenteral administration, etc.

The MSM, glucosamine, and PCE used in this invention can be employed in admixture with conventional excipients, i.e. pharmaceutically acceptable organic or inorganic carrier substances suitable for parenteral, enteral (e.g:, oral) application which do not deleteriously affect the active compound. Suitable pharmaceutically acceptable carriers include but are not limited to water, salt solutions, alcohols, gum arabic, vegetable oils, benzyl alcohols, polyethylene glycols, gelatine, carbohydrates such as lactose, amylose or starch, magnesium sterate, talc, silicic acid, viscous paraffin, perfume oil, fatty acid monoglycerides and diglycerides, pentaerythritol fatty acid esters, hydroxy methylcellulose, polyvinyl pyrrolidone, etc. The pharmaceutical preparations can be sterilized and, if desired, mixed with auxiliary agents such as, for example, lubricants, preservatives, stabilizers, wetting agents, emulsifiers, salts for influencing osmotic pressure, buffers, coloring agents, flavoring agents and/or aromatic substances and the like which do not deleteriously affect the active compound. Other pharmaceutically acceptable carriers include aqueous solutions, non toxic excipients, including salts, preservatives, buffers and the like, as described for instance, in *Remington's Pharmaceutical Sciences,* 18th ed. (1990), Mack Publishing Co., Easton, PA. The pH and exact concentration of the various components of the pharmaceutical composition are adjusted according to routine skills in the art. *See* Gilman et al. (eds.) (1990) *Goodman and Gilman's: The Pharmacological Bases of Therapeutics*, 8th ed., Pergamon Press. The MSM and/or PCE can also be combined where desired with other active agents, including PCE or MSM respectively.

The pharmaceutical compositions are preferably prepared and to be administered in dose units. Solid dose units are tablets, powders, capsules, granulated pellet forms and suppositories. For treatment of a subject, depending on activity of the compound, manner of administration, nature and severity of the disorder, age and body weight of the patient, different daily doses are necessary. Under certain circumstances, however, higher or lower daily doses may be appropriate. The average daily dosage of the compounds of this invention, are typically the following: for MSM, the average daily dose is generally about 1 to about 500 mg/kg/day, preferably about 20 to about 150 mg/kg/day, more preferably about 30 to about 90 mg/kg/day; for glucosamine or glucosamine sulfate, the average daily dose is generally about 1 to about 400 mg/kg/day, preferably about 10 to about 90 mg/kg/day, more preferably about 20 to about 40 mg/kg/day; for PCE, when the PCE comprises ground whole mussel, the average daily dose is generally about 1 to about 500 mg/kg/day, preferably about 10 to about 120 mg/kg/day, more preferably about 20 to about 60 mg/kg/day. For PCE components other than ground whole mussel, the average daily dose may vary but can be readily determined by one of skill in the art. The daily dosage may be administered as a single dose per day or as a plurality of divided doses per day. The daily dosage may also be administered on a non-daily basis, such as for example, every second or third day, provided an average daily dose as described above is achieved. The therapeutic administration of glucosamine is described in the following U.S. patents : U.S. Pat. No. 3,232,836 (Carlozzi); U.S. Pat. No. 3,682,076 (Rovati); U.S. Pat. No. 4,006,224 (Prudden); U.S. Pat. No. 5,364,845 (Henderson), U.S. Pat. No. 5,587363 (Henderson). The therapeutic administration of MSM is described in the following U.S. patents, U.S. Pat. No. 4,616,039 (Herschler); U.S. Pat. No. 4,863,748 (Herschler); U.S. Pat. No. 5,071,878 (Herschler). The therapeutic administration of PCE is described in the following U.S. Patents or patent applications, U.S. Patent Application publication No. US 2002/0018787 (Kendall and Lawson).

The combination of MSM, glucosamine, and PCE can be administered concurrently or alternately with other therapeutic treatments conventionally employed to treat the indicated diseases. For example, for the treatment of lupus erythmatosus, rheumatoid arthritis, and osteoarthritis, MSM, glucosamine, and PCE may be administered in conjunction with anti-inflammatory agents, including both corticosteroid agents such as, for example, prednisone or methylprednisolone and non-steroidal anti-inflammatory agents such as, for example, salicylates, carprofen, and ibuprofen. For the treatment of lupus erythmatosus, for example, the compounds of this invention may also be administered in conjunction with anti-malarial drugs including, for example, hydroxychloroquinone or in conjunction with cytotoxic chemotherapies including, for example, azathioprine and cyclophosphamide.

Another aspect of this invention is a kit containing a combination of MSM, glucosamine, and PCE formulations. The kit may contain all three components in separate formulations or any of the components combined into a single formulation. For example, a kit may contain a PCE formulation and a formulation comprising both MSM or glucosamine. Components of the kit may also be combined with other excipients or therapeutic agents. A kit would typically provide for an average daily dose of MSM, glucosamine, or PCE as described above.

It is also contemplated as an aspect of the invention that the Perna canaliculus/glucosamine/MSM combination be used in conjunction with manganese supplementation. Manganese is an essential cofactor in the production of cartilage and supplementation with manganese may enhance the efficacy of the inventive combination by guarding against manganese deficiency.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure.

### EXAMPLES

In the following examples, all parts and percentages are by weight unless otherwise indicated.

### Example 1 - Preparation of a therapeutically active PCE component

A therapeutically active formulation of *Perna canaliculus* is prepared by freeze-drying the flesh of the mussel and grinding it into a powder. The product can be formulated into capsules with the excipients of alfalfa, cellulose and magnesium stearate.

### Example 2 - Case Studies

The following specific case histories illustrate particular aspects of the invention.

The compositions of the present invention were administered to humans or small animals in tablet or capsule form. Specific products mentioned in the case histories are as follows:

| Product | Levels of Tablet/Capsule |
|---|---|
| Perna* | |
| Perna canaliculus | 500 mg |
| Alfalfa | 100 mg |

| Perna Plus* | |
|---|---|
| Perna canaliculus | 500 mg |
| Glucosamine Sulfate | 300 mg |
| Methylsulfonylmethane | 200 mg |

| Glyco-Flex* | |
|---|---|
| Perna canaliculus | 300 mg |
| Alfalfa | 100 mg |

| Glyco-Flex Plus* | |
|---|---|
| Perna canaliculus | 300 mg |
| Glucosamine HCl | 250 mg |
| Methylsulfonylmethane | 250 mg |

| Glyco-Flex Plus Double Strength* | |
|---|---|
| Perna canaliculus | 600 mg |
| Glucosamine HCl | 500 mg |
| Methylsulfonylmethane | 500 mg |

| | |
|---|---|
| * All of the above products are available from FoodScience Corp., Essex Junction, Vermont. | |

The following examples and case histories were conducted with humans or dogs. The unexpected faster response for relief of symptoms and improvement in recovery using the Pema canaliculus/glucosamine/methylsulfonylmethane combination of this invention as compared to using only products containing Perna canaliculus and alfalfa demonstrates the effectiveness of the invention. The products described above may have also included manganese to guard against manganese deficiency.

**CASE #1:** A 32 year old woman diagnosed with osteoarthritis in the left knee was administered 4 tablets/day of Perna product. After 3 weeks of treatment, she began to experience improvement: experiencing less pain, exhibiting reduced inflammation, and exhibiting greater mobility. After jogging, or with increased physical activity, however, pain and stiffness would return, even after two months of treatment with the Perna product. On rainy or humid days, the knee would become sensitive and painful. After 3 months on the Perna product, the individual switched from Perna to Perna Plus, at a dosage of 4 tablets/day. Perna Plus gave a noticeable improved response over that of the Perna after several days of treatment. Within seven days of commencing Perna Plus treatment, the sensitivity in the knee was significantly reduced and less pain was felt. After two weeks of treatment with Perna Plus, all pain and stiffness was eliminated, even after increased physical activity such as jogging. The individual continued to be free of pain while maintaining a dosage of 2-3 tablets/day of Perna Plus.

**CASE #2:** A 30 year old male dry wall installer reported extreme pain in his shoulder joints. The pain was thought to be related to his occupation. This individual's range of arm motion was limited, and he experienced soreness and stiffness every morning. Even after taking 6-8 Advil/day for relief, he still experienced pain and discomfort. On taking the Perna product, at a dosage of 6 capsules/day for several weeks, this individual experienced relief of pain in his shoulders, and he was able to reduce his use of Advil. His sleep quality improved and much of the soreness and stiffness was eliminated. One week after he discontinued taking the Perna product, the severe pain and soreness returned. On switching from the Perna product to the Perna Plus product, an improved response was found. After taking six tablets/day of Perna Plus for three days, the individual experienced a complete reversal of pain and no longer needed Advil for pain relief. The Perna Plus product worked faster and brought more complete relief of pain, soreness and morning stiffness as compared to the Perna product. The Perna Plus product helped restore a more complete range of motion for both his arms without discomfort. The use of the Perna product by itself was not able to accomplish this.

**CASE #3:** Amber, a seven year old female spayed Labrador Retriever, was diagnosed with chondrodysplasia. The dog had developed significant degenerative joint disease by age three. Rimadyl (Carprofen) was administered to control the pain and other symptoms. For six months, Glyco-Flex DS was administered at a dosage of 2 tablets in the morning and 2 tablets in the afternoon. There was no rapid change in clinical condition, but the product brought some improvement in the dog's level of activity. The dog was then switched to Glyco-Flex Plus DS and the Rimadyl was continued. Within 10 to 12 days of treatment with the Glyco-Flex Plus DS, Amber became much more active, climbing stairs with ease, running with other dogs, and showing greater ease in jumping. After treatment with Glyco-Flex Plus DS over time, the dosage of Rimadyl was cut back; this reduction in Rimadyl dosage could not be accomplished with Glyco-Flex treatment. Glyco-Flex Plus treatment was effective in reducing pain, reducing inflammation, and increasing physical movement, while at the same time allowing for a safer treatment option by reducing the levels of Rimadyl that were required to maintain the animal.

**CASE #4:** Two geriatric Doberman Pinchers with arthritis in the hips, who had been previously on Glyco-Flex, experienced significant improvement when treatment with Glyco-Flex Plus was substituted for treatment with Glyco-Flex. The animals had previously been treated with 4 tablets/day of Glyco-Flex for several months. This treatment resulted in some reduction of symptoms, including reduction in pain and inflammation and an increase in range of motion. After switching from treatment with Glyco-Flex to treatment with 4 tablets/day of Glyco-Flex Plus, a greater improvement in terms of increased range of movement and reduction of pain and joint stiffness was observed in both animals. The animals had been given 75 mg/day of Rimadyl (Carprofen) to control pain. Treatment with Glyco-Flex Plus allowed for a reduction in Rimadyl administration to twice weekly, with the maintenance of adequate pain management. No other changes in the dogs treatment program were implemented, and the observed improvements in the dogs was attributed to the use of Glyco-Flex Plus.

**CASE #5:** A veterinarian has reported a faster healing response in arthritic dogs when the dogs were treated with Glyco-Flex Plus as compared to treatment with Glyco-Flex. Several dogs undergoing treatment with Glyco-Flex Plus were compared to other comparable cases where Glyco-Flex treatment was used. The dogs owners reported significant and discernable improvement with treatment with Glyco-Flex Plus, including increased pain relief, greater flexing and ease of movement, and less lameness as compared to treatment with Glyco-Flex. Clinical examination showed improved palpation sensitivity scores and reduction trotting lameness with Glyco-Flex Plus treatment versus Glyco-Flex treatment. The use of pain killers and anti-inflammatory drugs (Rimadyl, Ibuprofen) was generally cut back by more than 70% in animals treated with Glyco-Flex Plus, allowing for more rapid rebuilding of the damaged joints. These studies indicated that the synergistic combination of the Perna canaliculus, Glucosamine and MSM contributed to a more rapid improvement in the comfort levels and functional levels of dogs that were changed from treatment with Glyco Flex to Glyco Flex Plus. According to the reports of the veterinarian, Glyco-Flex Plus treatment provided a more immediate response and greatly improved the quality of life for dogs with degenerative joint disease.

**CASE #6:** A 44 year old human female long distance runner developed knee problems, including loss of cartilage and inflammation in the tissues around the knees. In addition, she was diagnosed as having bursitis in the hip area. Symptoms included knee and hip pain, stiffness in the morning, and extreme discomfort while exercising or walking down stairs. Range of motion was extremely limited in the knees.

This individual started taking 4 tablets/day of the Perna product, and after 3 weeks of treatment, she began to receive some relief from the pain, although the pain was never eliminated. After 6 months of treatment with the Perna product, the individual began to take the Perna Plus product instead of the Perna product. After treatment with the Perna Plus product, she experienced a dramatic improvement in symptoms in comparison to treatment with the Perna product. Within a few weeks of commencing an 8 tablet/day Perna Plus treatment, the pain in her knees largely disappeared and greater movement and flexibility was noted, with significantly less inflammation. She no longer experienced pain in going down stairs or bursitis of the hip. A maintenance dose of 4 tablets/day allowed for the maintenance of this same level of comfort, relief, and exercise tolerance.

**CASE #7:** Ebby Wags, a seven year old, overweight, mixed breed female dog was diagnosed with a lame, sore right front leg. She exhibited increased pain and discomfort upon flexing or while exercising. The range of motion in this leg was slightly restricted. After 7 to 10 days of treatment with 2 tablets/day of Glyco-Flex Plus DS, the dog was observed by the owner to be walking better, limping less, and to begin running and playing more. After 3 weeks of treatment with Glyco-Flex Plus DS, a veterinary examination indicated that the dog's standing lameness, trotting lameness and palpitation sensitivity scores had nearly normalized. The dog was experiencing very little pain, had a better attitude, and exhibited increased exercise tolerance. Range of motion in the affected joint was increased to 95%. After 6 weeks of treatment with Glyco-Flex Plus DS, the owner reported that the dog runs much better and gets up easier, and that the joint problem showed much improvement in pain level. According to the veterinarian, this example demonstrates a fairly fast response time (7-10 days) for the combination product (Glyco-Flex Plus PS) to effect a reduction in lameness, pain and inflammation as compared to the Glyco-Flex product (which contained only Perna canaliculus and alfalfa). The Glyco-Flex product generally requires treatment for up to 3 to 4 weeks to give a response.

Based upon field testing by veterinarians, one of the key advantages of a combination product of the present invention Perna canaliculus/Glucosamine/MSM is that the use of steroids and NSAIDS can be reduced significantly or eliminated from the anti-arthritic protocol. In acute cases, it has been demonstrated that the use of the Perna canaliculus/Glucosamine/MSM combination in conjunction with Rimadyl (Carprofen) for control of pain, the dosage of the drug can be curtailed significantly within 7 to 10 days, as the combination product begins to take effect in reducing pain and inflammation. Recent reports reveal that, for many dogs, Rimadyl can result in serious liver disease and even death if used in high doses over too long of a period of time.

Thus it can be seen that the combination of the present invention of Perna canaliculus, Glucosamine and methylsulfonylmethane (MSM) unexpectedly provides an enhanced effect in reducing pain and inflammation associated with joint and connective tissue conditions.

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

From the foregoing description, one skilled in the art can easily ascertain the essential characteristics of this invention and, can make various changes and modifications of the invention to adapt it to various usages and conditions.

## Claims

1. Use of a methylsulfonylmethane component, a glucosamine component, and at least one Perna canaticulus component for the manufacture of a medicament for the treatment of an inflammatory disease.

2. Use according to claim 1, wherein the inflammatory disease is an autoimmune disease.

3. Use according to claim 2, wherein the autoimmune disease is lupus erythematosus.

4. Use according to claim 3, wherein at least one Perna canaliculus component is freeze-dried ground whole mussel.

5. Use of a methylsulfonylmethane component, a glucosamine component, and at least one Perna canaliculus component for the manufacture of a medicament for the treatment of an existing or potential connective tissue condition.

6. Use according to claim 5, wherein the connective tissue condition is osteoarthritis.

7. Use according to claim 5, wherein the connective tissue condition is rheumatoid arthritis.

8. A composition comprising a methylsulfonylmethane component, a glucosamine component, and at least one Perna canaliculus component.

9. A composition according to claim 8, wherein at least one Perna canaliculus component comprises freeze-dried ground whole mussel.

10. A composition according to claim 8 which is suitable for use as a dietary supplement.

11. A kit comprising a methylsulfonylmethane formulation, a glucosamine formulation, and a Perna canaliculus formulation, wherein the Perna canaliculus formulation comprises at least one Perna canaliculus component.

12. A kit according to claim 11, wherein the methylsulfonylmethane formulation, the glucosamine and the Perna canaliculus formulation are suitable for use as dietary supplements via oral administration.

13. A kit according to claim 12, wherein at least one Perna canaliculus component comprises ground freeze-dried whole mussel.

14. Use according to claim 1 or 5, further comprising manganese.

15. A composition according to claim 8, further comprising manganese.

16. A kit according to claims 11, further comprising manganese.

17. Use according to claim 1 or 5, wherein the medicament is provided for administration to a human.

18. Use according to claim 1 or 5, wherein the medicament is provided for administration to a non-human mammal.

## Patentansprüche

1. Verwendung einer Methylsulfonylmethan-Komponente, einer Glucosamin-Komponente und mindestens einer Perna canaliculus-Komponente zur Herstellung eines Medikaments zur Behandlung einer Entzündungskrankheit.

2. Verwendung nach Anspruch 1, worin die Entzündungskrankheit eine Autoimmunerkrankung ist.

3. Verwendung nach Anspruch 2, worin die Autoimmunerkrankung Lupus erythematosus ist.

4. Verwendung nach Anspruch 3, worin mindestens eine Perna canaliculus-Komponente eine gefriergetrocknet gemahlene Gesamtmuschel ist.

5. Verwendung einer Methylsulfonylmethankomponente einer Glucosamin-Komponente und mindestens einer Perna canaliculus-Komponente zur Herstellung eines Medikaments zur Behandlung einer bestehenden oder potentiellen Bindegewebeerkrankung.

6. Verwendung nach Anspruch 5, worin die Bindegewebeerkrankung Osteoarthritis ist.

7. Verwendung nach Anspruch 5, worin die Bindegewebeerkrankung rheumatoide Arthritis ist.

8. Zusammensetzung, umfassend eine Methylsulfonylmethankomponente, eine Glucosaminkomponente und mindestens eine Perna canaliculus-Komponente.

9. Zusammensetzung nach Anspruch 8, worin mindestens eine Pema canaliculus-Komponente gefriergetrocknet gemahlene Gesamtmuschel umfasst.

10. Zusammensetzung nach Anspruch 8, die geeignet ist als eine Dietergänzung.

11. Kit, umfassend eine Methylsulfonylmethan-Formulierung, eine Glucosamin-Formulierung und mindestens eine Perna canaliculus-Formulierung, worin die Pema canalicus-Formulierung mindestens eine Perna canaliculus-Komponente umfasst.

12. Kit nach Anspruch 11, worin die Methylsulfonylmethan-Formulierung, die Glucosamin- und die Perna canaliculus-Formulierung zur Verwendung als Dietergänzungen durch orale Verabreichung geeignet sind.

13. Kit nach Anspruch 12, worin mindestens eine Perna canaliculus-Komponente gefriergetrocknet gemahlene Gesamtmuschel umfasst.

14. Verwendung nach Anspruch 1 oder 5, weiterhin umfassend Mangan.

15. Zusammensetzung nach Anspruch 8, weiterhin umfassend Mangan.

16. Kit nach Anspruch 11, weiterhin umfassend Mangan.

17. Verwendung nach Anspruch 1 oder 5, worin das Medikament zur Verabreichung an einen Menschen bereitgestellt wird.

18. Verwendung nach Anspruch 1 oder 5, worin das Medikament zur Verabreichung an einen nichtmenschlichen Säuger bereitgestellt wird.

## Revendications

1. Utilisation d'un composant de méthylsulfonylméthane, d'un composant de glucosamine et d'au moins un composant de *Perna canaliculus* pour la préparation d'un médicament destiné au traitement d'une maladie inflammatoire.

2. Utilisation selon la revendication 1, dans laquelle la maladie inflammatoire est une maladie auto-immune.

3. Utilisation selon la revendication 2, dans laquelle la maladie auto-immune est un lupus érythémateux.

4. Utilisation selon la revendication 3, dans laquelle au moins un composant de *Perna canaliculus* est des moules entières broyées et lyophilisées.

5. Utilisation d'un composant de méthylsulfonylméthane, d'un composant de glucosamine et d'au moins un composant de *Perna canaliculus* pour la préparation d'un médicament destiné au traitement d'un état du tissu conjonctif existant ou potentiel.

6. Utilisation selon la revendication 5, dans laquelle l'état du tissu conjonctif est l'arthrose.

7. Utilisation selon la revendication 5, dans laquelle l'état du tissu conjonctif est la polyarthrite rhumatoïde.

8. Composition comprenant un composant de méthylsulfonylméthane, un composant de glucosamine et au moins un composant de *Perna canaliculus*.

9. Composition selon la revendication 8, dans laquelle au moins un composant de *Perna canaliculus* comprend des moules entières broyées et lyophilisées.

10. Composition selon la revendication 8, qui est appropriée à une utilisation comme complément alimentaire.

11. Kit comprenant une formulation de méthylsulfonylméthane, une formulation de glucosamine et une formulation de *Perna canaliculus*, la formulation de *Perna canaliculus* comprenant au moins un composant de *Perna canaliculus*.

12. Kit selon la revendication 11, dans laquelle la formulation de méthylsulfonylméthane, la glucosamine et la formulation de *Perna canaliculus* sont appropriées à une utilisation comme compléments alimentaires par administration orale.

13. Kit selon la revendication 12, dans laquelle au moins un composant de *Perna canaliculus* comprend des moules entières broyées et lyophilisées.

14. Utilisation selon la revendication 1 ou 5, comprenant en outre du manganèse.

15. Composition selon la revendication 8, comprenant en outre du manganèse.

16. Kit selon la revendication 11, comprenant en outre du manganèse.

17. Utilisation selon la revendication 1 ou 5, dans laquelle le médicament est prévu pour une administration à l'être humain.

18. Utilisation selon la revendication 1 ou 5, dans laquelle le médicament est prévu pour une admnistration à un mammifère non humain.
